# EUROPÄISCHE PATENTANMELDUNG

(11) **EP 4 116 704 A1**
(43) Veröffentlichungstag der Anmeldung: **11.01.2023**
(21) Anmeldenummer: 22181505.3
(22) Anmeldetag: 28.06.2022
(51) Int. Cl.: G01N 21/84, G01J 3/28, G01N 21/47, G01N 21/31, A01B 79/02, G01N 33/00, G01N 33/02, G01N 21/25

(54) **VERFAHREN UND VORRICHTUNG ZUM ERKENNEN EINES PFLANZENGESUNDHEITSZUSTANDS VON PFLANZEN FÜR EINE LANDMASCHINE**

(30) Priorität: 05.07.2021 DE 102021207009
(71) Anmelder: Robert Bosch GmbH, 70442 Stuttgart (DE)
(72) Erfinder: Koenig, Jens, 71706 Markgroeningen (DE); Houis, Nicolas, 74321 Bietigheim-Bissingen (DE)

(57) **Zusammenfassung**

Ein Verfahren zum Erkennen eines Pflanzengesundheitszustands von Pflanzen (105) für eine Landmaschine umfasst einen Schritt des Einlesens eines Bildsignals (125), das einen zumindest einen Teilbereich (130) umfassenden Interessenbereich (140) abbildet, wobei dem Interessenbereich (140) eine geringe spektrale Auflösung aufweisende Bilddaten zugewiesen sind. In einem Schritt des Empfangens wird ein Spektrumsignal (145) empfangen, das den Teilbereich (130) des Interessenbereichs (140) durch eine hohe spektrale Auflösung aufweisende Spektraldaten abbildet. In einem Schritt des Bestimmens wird ein Datensatz (150) bestimmt, der zumindest einen von dem Teilbereich (130) umfassten Pflanzenbereich (151) abbildet, wobei dem Pflanzenbereich (151) eine gegenüber der geringen spektralen Auflösung modifizierte spektrale Auflösung aufweisende Schätzdaten zugewiesen sind, unter Verwendung des Bildsignals (125) und des Spektrumsignals (145).

## Beschreibung

### Stand der Technik

Der Ansatz geht von einer Vorrichtung oder einem Verfahren nach Gattung der unabhängigen Ansprüche aus. Gegenstand des vorliegenden Ansatzes ist auch ein Computerprogramm.

Die DE 10 2011 003 647 A1 offenbart eine Messeinrichtung zur Bestimmung eines Vegetationsindex-Werts. Die US 6 160 902 A offenbart ein Verfahren zur Überwachung des Stickstoffzustandes unter Verwendung eines multispektralen Abbildungssystems.

### Offenbarung der Erfindung

Vor diesem Hintergrund werden mit dem hier vorgestellten Ansatz ein Verfahren zum Erkennen eines Pflanzengesundheitszustands von Pflanzen für eine Landmaschine, weiterhin eine Vorrichtung, die dieses Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Die mit dem vorgestellten Ansatz erreichbaren Vorteile bestehen darin, dass eine Möglichkeit geschaffen wird, schnell und einfach einen Gesundheitszustand einer Pflanze zu erkennen, um beispielsweise eine geeignete Behandlung der Pflanze durch eine Landmaschine zu ermöglichen.

Es wird ein Verfahren zum Erkennen eines Pflanzengesundheitszustands von Pflanzen für eine Landmaschine vorgestellt. Das Verfahren umfasst einen Schritt des Einlesens, einen Schritt des Empfangens und einen Schritt des Bestimmens. Im Schritt des Einlesens wird ein Bildsignal eingelesen, das einen zumindest einen Teilbereich umfassenden Interessenbereich abbildet, wobei dem Interessenbereich eine geringe spektrale Auflösung aufweisende Bilddaten zugewiesen sind. Im Schritt des Empfangens wird ein Spektrumsignal empfangen, das den Teilbereich des Interessenbereichs durch eine hohe spektrale Auflösung aufweisende Spektraldaten abbildet. Im Schritt des Bestimmens wird ein Datensatz bestimmt, der zumindest einen von dem Teilbereich umfassten Pflanzenbereich abbildet, wobei dem Pflanzenbereich abbildet, wobei dem Pflanzenbereich eine gegenüber der geringen spektralen Auflösung modifizierte spektrale Auflösung aufweisende Schätzdaten zugewiesen sind, unter Verwendung des Bildsignals und des Spektrumsignals.

Dieses Verfahren kann beispielsweise in Software oder Hardware oder in einer Mischform aus Software und Hardware beispielsweise in einem Steuergerät implementiert sein.

Der Interessenbereich wird im Englischen auch "region of interest", kurz "ROI", genannt. Bei dem Interessenbereich kann es sich um einen von einer oder mehreren Pflanzen bewachsenen Bereich handeln, der von einer Sprüh- und Streuvorrichtung der Landmaschine behandelt wird oder werden soll. Die Sprüh- und Streuvorrichtung kann beispielsweise Feldspritzen und/oder Düngestreuer aufweisen. Das Bildsignal repräsentiert eine Abbildung des gesamten Interessenbereichs und weist gemäß einer Ausführungsform eine hohe örtliche Auflösung auf. Das Spektrumsignal hingegen repräsentiert lediglich eine Abbildung eines oder mehrerer Teilbereiche des Interessenbereichs und weist gemäß einer Ausführungsform gegenüber dem Bildsignal eine geringere örtliche Auflösung auf. Die Bilddaten mit der geringen spektralen Auflösung weisen bevorzugt zwei bis vier, weiter bevorzugt zwei oder drei Wellenlängenbereiche auf. Die Bilddaten mit der geringen spektralen Auflösung können einen ersten Wellenlängenbereich und zumindest einen zweiten Wellenlängenbereich abbilden, wobei sich der erste Wellenlängenbereich von dem zweiten Wellenlängenbereich unterscheidet. Die Bilddaten können den unterschiedlichen Wellenlängenbereichen zugeordnete Intensitätswerte umfassen. Der erste Wellenlängenbereich und der zweite Wellenlängenbereich umfassen Wellenlängenbereiche, welche von Pflanzen reflektiert werden. Die geringe spektrale Auflösung ermöglicht typischerweise keine Erkennung einer kranken Pflanze oder Erkennung einer spezifischen Pflanzenkrankheit oder eines Nährstoffmangels. Der erste Wellenlängenbereich kann beispielsweise einen roten Spektralbereich von 630nm bis 680nm umfassen. Der zweite Wellenlängenbereich kann einen nahen Infrarotspektralbereich von 730nm bis 1100nm umfassen. Die Bilddaten mit der geringen spektralen Auflösung können ferner auch einen dritten Wellenlängenbereich und/oder vierten Wellenlängenbereich abbilden, wobei sich der erste Wellenlängenbereich und der zweite Wellenlängenbereich von dem dritten und vierten Wellenlängenbereich unterscheiden. Die hohe spektrale Auflösung der Spektraldaten kann höher sein als die geringe spektrale Auflösung der Bilddaten. Die Spektraldaten können somit einer Mehrzahl von Wellenlängenbereichen zugeordnete Intensitätswerte umfassen. Die Spektraldaten mit der hohen spektralen Auflösung weisen bevorzugt vier bis acht oder mehr als acht Wellenlängenbereiche auf. Hierbei weisen die Spektraldaten mit der hohen spektralen Auflösung bevorzugt zumindest zwei der Wellenlängenbereiche der Bilddaten mit der geringen spektralen Auflösung und zumindest zwei weitere Wellenlängenbereiche auf. D.h., mit anderen Worten, dass sich bevorzugt zumindest oder genau zwei Wellenlängenbereiche überschneiden und ferner zumindest oder genau zwei zusätzliche Wellenlängenbereiche vorgesehen sind. Die beiden zusätzlichen Wellenlängenbereiche der Spektraldaten mit der hohen spektralen Auflösung umfassen bevorzugt Wellenlängen in einem Bereich von 630nm bis 1100nm. Die unter Verwendung des vorgestellten Verfahrens modifizierte spektrale Auflösung der Schätzdaten kann höher sein als die geringe spektrale Auflösung der Bilddaten oder sogar die hohe spektrale Auflösung der Spektraldaten aufweisen. Unter Verwendung des hier vorgestellten Verfahrens kann demnach vorteilhafterweise ein Datensatz erzeugt werden, der die hohe örtliche Auflösung des Bildsignals aufweist und zumindest für einen oder mehrere Teilbereiche eine gegenüber dem Bildsignal verbesserte spektrale Auflösung aufweist, um den Pflanzengesundheitszustand der Pflanze erkennbar zu machen.

Im Schritt des Bestimmens kann der Datensatz des Pflanzenbereichs unter Verwendung zumindest einer Transferfunktion aus dem Bildsignal und dem Spektrumsignal bestimmt werden. Die Transferfunktion kann als hinterlegte, beispielsweise zuvor bestimmte, Transferfunktion eingelesen und/oder angewandt werden. Unter Verwendung einer solchen Transferfunktion können die Bilddaten des Teilbereichs und die entsprechenden Spektraldaten desselben Teilbereichs einander zugeordnet werden. Es kann ferner auch eine Interpolation und Extrapolation durchgeführt werden, um beispielsweise weitere Schätzdaten für jene Teilbereiche des Interessenbereichs zu erhalten, für welche kein Spektrumsignal zur Verfügung steht.

Beispielsweise kann im Schritt des Bestimmens unter Verwendung der zumindest einen Transferfunktion ein von den Bilddaten für den Pflanzenbereich abgebildetes Spektrum um zumindest einen Spektralbereich eines von den Spektraldaten für den Pflanzenbereich abgebildeten Spektralbereich ergänzt werden.

Im Schritt des Bestimmens können die Spektraldaten zu den Bilddaten des Teilbereichs unter Verwendung einer örtlichen Transferfunktion referenziert werden, um eine räumliche Zuordnung zwischen den Spektraldaten und den Bilddaten zu erhalten. Eine solche räumliche Zuordnung ermöglicht es, eine höhere räumliche Auflösung der Bilddaten mit der niedrigeren räumlichen Auflösung der Spektraldaten zu erhalten.

Ferner können im Schritt des Bestimmens die Spektraldaten zu den Bilddaten referenziert werden, indem Bildintensitäten der Spektraldaten zu Bildintensitäten der Bilddaten referenziert werden. Hierbei können beispielsweise im Schritt des Bestimmens Bildintensitäten einer bestimmten Wellenlänge oder einer Kombination aus Intensitäten einer Mehrzahl von Wellenlängen der Spektraldaten zu Bildintensitäten einer bestimmten Wellenlänge oder einer Kombination aus Intensitäten einer Mehrzahl von Wellenlängen der Bilddaten referenziert werden.

Zusätzlich oder alternativ können im Schritt des Bestimmens die Spektraldaten zu den Bilddaten unter Verwendung einer spektralen Transferfunktion referenziert werden, um eine spektrale Zuordnung zwischen den Spektraldaten und den Bilddaten zu erhalten. Eine solche spektrale Zuordnung ermöglicht es, eine niedrigere spektrale Auflösung der Bilddaten mit der höheren spektralen Auflösung der Spektraldaten zu erhalten.

Beispielsweise können im Schritt des Bestimmens die Spektraldaten zu den Bilddaten unter Verwendung zumindest einer Spektrallinie referenziert werden, die sowohl von den Spektraldaten als auch von den Bilddaten abgebildet wird. Zusammengefasst bedeutet dies, dass zwei Algorithmen/Transferfunktionen angewandt werden können: bei Anwendung der örtlichen Transferfunktion erfolgt eine Referenzierung der zwei örtlichen Bildintensitäten zueinander, also Bild zu Bild, z. B. anhand Pflanzen/Blattkonturen und/oder bei Anwendung der spektralen Transferfunktion erfolgt eine Referenzierung der zwei spektralen Datensätze zueinander, also Spektrum zu Spektrum, anhand der zwei oder drei überlappenden Spektralbereiche für jedes Pixel (cluster) in dem zuvor örtlich zueinander referenzierten Bereich. Dabei werden die Fit-Parameter der Transferfunktion erhalten.

Es ist weiterhin von Vorteil, wenn das Verfahren gemäß einer Ausführungsform einen Schritt des Identifizierens aufweist, in dem der Pflanzengesundheitszustand der Pflanze in dem Interessenbereich unter Verwendung des Datensatzes identifiziert wird.

Im Schritt des Identifizierens kann der Pflanzengesundheitszustand ferner unter Verwendung von Pflanzenreferenzdaten identifiziert werden. Beispielsweise kann im Schritt des Identifizierens der Pflanzengesundheitszustand der Pflanze im Interessenbereich unter Verwendung der Pflanzenreferenzdaten identifiziert werden, die Pflanzenreflexionsspektren für bestimmte Pflanzen und/oder Pflanzengesundheitszustände umfassen. Beispielsweise kann hierbei eine Nachschlagetabelle mit hinterlegten Pflanzenreflexionsspektren für bestimmte Pflanzen und zugeordneten Pflanzengesundheitszuständen verwendet werden, um den Pflanzengesundheitszustand schnell und einfach durch Zuordnung zu identifizieren.

Es ist weiterhin von Vorteil, wenn im Schritt des Identifizierens eine Blattkontur und/oder Farbverteilung der Pflanze unter Verwendung des Bildsignals erkannt wird. So kann im Schritt des Identifizierens der Pflanzengesundheitszustand der Pflanze ferner unter Verwendung der Blattkontur und/oder Farbverteilung der Pflanze identifiziert werden.

Das Verfahren kann weiterhin einen Schritt des Erzeugens aufweisen, in dem unter Verwendung des Datensatzes ein Einstellsignal zum Einstellen einer Art und/oder Dosierung eines Sprühmittels und/oder Streumittels einer Sprüh- und Streuvorrichtung zum Behandeln des Interessenbereichs erzeugt wird. Im Schritt des Erzeugens kann das Einstellsignal ferner unter Verwendung des im Schritt des Identifizierens identifizierten Pflanzengesundheitszustands erzeugt werden. Durch das Einstellsignal können Eingaben optimiert werden, um den besten oder einen vordefinierten Pflanzengesundheitszustand zu bewirken. Beispielsweise kann durch das Einstellsignal eine Ausgabe eines geeigneten Düngemittels für eine Pflanze eingestellt werden, für welche im Schritt des Identifizierens ein bestimmter Nährstoffmangel identifiziert wurde. So ist bei Mais und vielen anderen Pflanzen ein Stickstoffmangel beispielsweise durch eine hellgrüne oder gelbe Blattfarbe zu erkennen, ein Phosphormangel zeigt sich durch eine rötlich bis purpurfarbene Blattverfärbung, ein Kaliummangel zeigt sich durch trockene Blattränder, ein Magnesiummangel ist durch weiße Streifen entlang der Blattadern erkennbar. Verbrannte Blattspitzen und -ecken sowie weiße Verfärbungen deuten auf Herbizidschäden hin, graugrüne sowie eingerollte Blätter deuten auf Trockenstress hin, wohingegen kleine Flecken auf Krankheiten wie die Blattfleckenkrankheit hinweisen. Gesunde Maisblätter weisen hingegen eine dunkelgrüne Blattfarbe auf.

Es ist weiterhin von Vorteil, wenn das Verfahren gemäß einer Ausführungsform einen Schritt des Ausgebens aufweist, in dem unter Verwendung der Sprüh- und Streuvorrichtung und/oder des Einstellsignals die im Schritt Erzeugens eingestellte Art und/oder Dosierung des Sprühmittel und/oder Streumittels ausgegeben wird. So kann der Pflanzengesundheitszustand der Pflanze/n direkt oder bei zukünftigen Behandlungen bzw. Besprühungen, Bestreuungen, optimiert werden.

Das Verfahren kann ferner einen Schritt des Erfassens aufweisen, in dem das Spektrumsignal unter Verwendung einer Spektraleinrichtung mit zumindest einer hyperspektralen oder multispektralen Bildgebungskamera, einem Spektrometer und/oder einer Mehrzahl von LEDs mit Fotodioden erfasst wird. Das Spektrumsignal kann ein von der Spektraleinrichtung aufgenommenes Bild oder Spektrum des Teilbereichs repräsentieren. Die Spektraldaten können beispielsweise Intensitätswerte der einzelnen Bildpunkte eines unter Verwendung der Spektraleinrichtung aufgenommenen Bilds repräsentieren.

Die Spektraleinrichtung kann an einem Spritzgestänge der Sprüh- und Streuvorrichtung angeordnet oder anordenbar sein.

Im Schritt des Einlesens kann das Bildsignal von einer Schnittstelle zu einer Kameraeinrichtung mit einer ersten Kamera und/oder zumindest einer zweiten Kamera eingelesen werden, wobei das Bildsignal ein von der ersten Kamera aufgenommenes erstes Kamerabild und/oder ein von der zweiten Kamera aufgenommenes zweites Kamerabild repräsentiert. Die Bilddaten können beispielsweise Intensitätswerte der einzelnen Bildpunkte eines unter Verwendung der Kameraeinrichtung aufgenommenen Bilds repräsentieren. Die Kameraeinrichtung kann an einem Spritzgestänge der Landmaschine angeordnet oder anordenbar sein. Die Kameraeinrichtung kann beispielsweise einen optischen Filter aufweisen, der lediglich Wellenlängen in einem roten Bandbereich, beispielsweise von 630nm bis 680nm, und/oder infraroten oder nahinfraroten Bandbereich, beispielsweise von 730nm bis 1100nm, durchlässt. So können in dem Interessenbereich Pflanzen, welche Wellenlängen in dem roten/infraroten/nahinfraroten Bandbereich von 630nm bis 680nm und/oder 730nm bis 1100nm reflektieren, erkannt werden.

Im Schritt des Empfangens kann zumindest ein zweites Spektrumsignal empfangen werden, das einen sich von dem Teilbereich unterscheidenden zweiten Teilbereich der Teilbereiche des Interessenbereichs durch eine hohe spektrale Auflösung aufweisende Spektraldaten abbildet, wobei im Schritt des Bestimmens der Datensatz unter Verwendung ferner des zweiten Spektrumsignals bestimmt wird. Unter Verwendung mehrerer Spektrumsignale können mehrere Teilbereiche des Interessenbereichs hinsichtlich eines Pflanzengesundheitszustands von darin angeordneten Pflanzen überprüft werden.

Das Verfahren kann ferner einen Schritt des Empfangens eines Referenzsignals von einem Referenzsensor aufweisen, um die Transferfunktion zu bestimmen. Ein solches Referenzsignal kann zur Analyse und Berücksichtigung der Intensität, der räumlichen und winkelmäßigen Verteilung, der spektralen Verteilung des Umgebungslichts, z. B. des Sonnenlichts, in der Transferfunktion und Bilderkennung der als hyperspektrale oder multispektrale Bildgebungskamera ausgeformten Spektraleinrichtung dienen. Der Referenzsensor kann z. B. auf einem Kabinendach eines landwirtschaftlichen Geräts/Schleppers, welches/r die Sprüh- und Streuvorrichtung aufweist, oder an einer Düse der Sprüh- und Streuvorrichtung angeordnet sein.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante des Ansatzes in Form einer Vorrichtung kann die dem Ansatz zugrunde liegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EEPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:
- Fig. 1: eine schematische Darstellung einer Vorrichtung gemäß einem Ausführungsbeispiel zum Erkennen eines Pflanzengesundheitszustands von Pflanzen für eine Landmaschine;
- Fig. 2: eine schematische Darstellung einer Vorrichtung gemäß einem Ausführungsbeispiel;
- Fig. 3: eine schematische Darstellung von Bilddaten und Spektraldaten zur Verwendung mit einer Vorrichtung gemäß einem Ausführungsbeispiel;
- Fig. 4: ein Ablaufdiagramm eines Verfahrens zum Erkennen eines Pflanzengesundheitszustands von Pflanzen für eine Landmaschine gemäß einem Ausführungsbeispiel; und
- Fig. 5: Spektralsignaturen von gesunden Blättern und kranken Blättern einer Rübenpflanze; und
- Fig. 6: eine schematische Darstellung einer Landmaschine mit einer Vorrichtung gemäß einem Ausführungsbeispiel.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

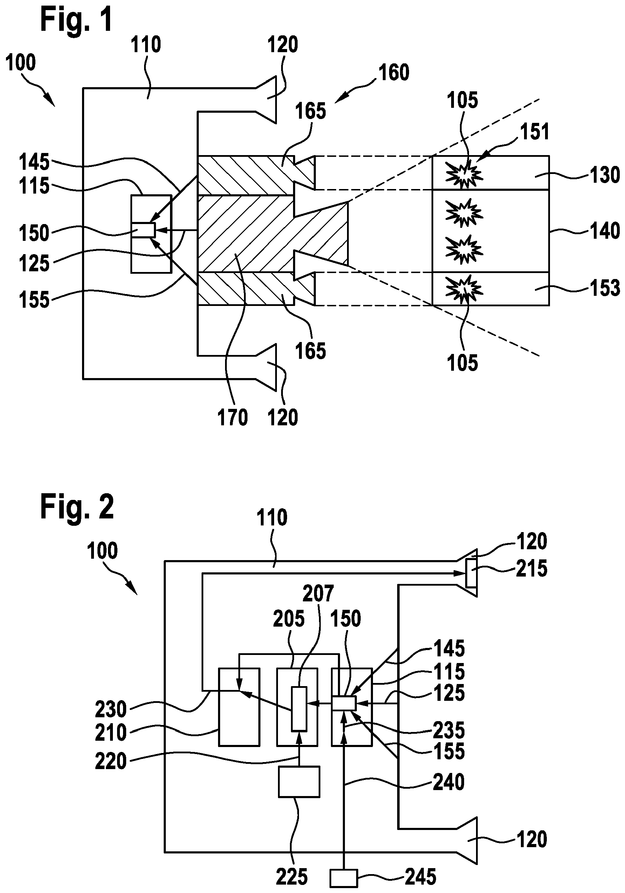

Fig. 1 zeigt eine schematische Darstellung einer Vorrichtung 100 gemäß einem Ausführungsbeispiel zum Erkennen eines Pflanzengesundheitszustands von Pflanzen 105 für eine Landmaschine mit hier beispielhaft einer Sprüh- und Streuvorrichtung 110.

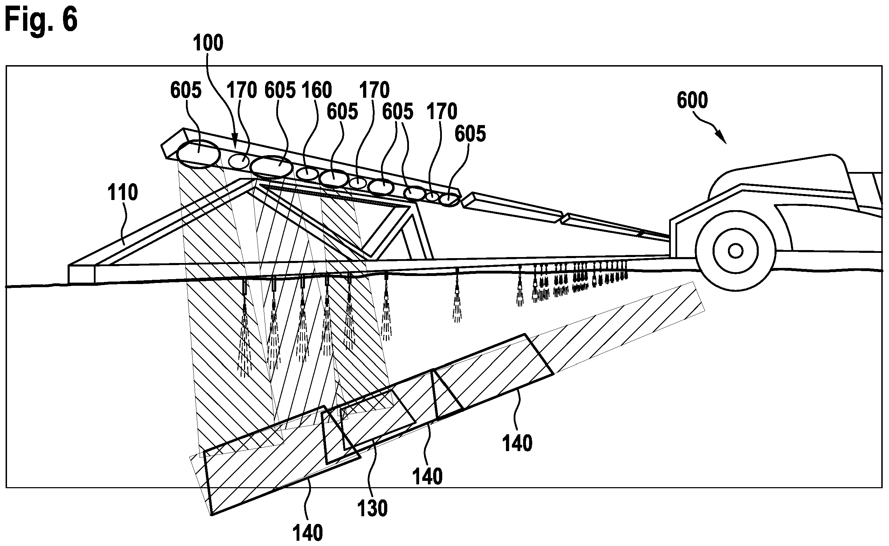

Die Vorrichtung 100 weist zumindest eine Bestimmeinrichtung 115 auf, die gemäß diesem Ausführungsbeispiel lediglich beispielhaft in oder an der Sprüh- und Sprüh- und Streuvorrichtung 110 angeordnet ist. Die Sprüh- und Streuvorrichtung 110 ist zur Verwendung an der in Fig. 6 gezeigten Landmaschine ausgeformt, die ein landwirtschaftliches Gerät/Fahrzeug sein kann. Die Sprüh- und Streuvorrichtung 110 ist zur Ausgabe eines Sprühmittels und/oder Streumittels auf beispielsweise eine von Pflanzen 105 bewachsene Agrarfläche ausgebildet. Die Sprüh- und Streuvorrichtung 110 weist hierzu gemäß diesem Ausführungsbeispiel einen oder mehrere Düsen 120 zum Ausgeben des Sprühmittels und/oder Streumittels auf. Gemäß einem alternativen Ausführungsbeispiel ist die Vorrichtung 100 an einer beliebigen Stelle der Landmaschine angeordnet.

Die Bestimmeinrichtung 115 ist ausgebildet, um ein Bildsignal 125 einzulesen, das einen zumindest einen Teilbereich 130 umfassenden Interessenbereich 140 abbildet, wobei dem Interessenbereich 140 eine geringe spektrale Auflösung aufweisende Bilddaten zugewiesen sind. Die Bestimmeinrichtung 115 ist weiterhin ausgebildet, um ein Spektrumsignal 145 zu empfangen, das den Teilbereich 130 des Interessenbereichs 140 durch eine hohe spektrale Auflösung aufweisende Spektraldaten abbildet. Die Bestimmeinrichtung 115 ist ausgebildet, um unter Verwendung des Bildsignals 125 und des Spektrumsignals 145 einen Datensatz 150 zu bestimmen, der zumindest einen von dem Teilbereich 130 umfassten Pflanzenbereich 151 mit zumindest einer der Pflanzen 105 abbildet, wobei dem Pflanzenbereich 151 eine gegenüber der geringen spektralen Auflösung modifizierte spektrale Auflösung aufweisende Schätzdaten zugewiesen sind. Lediglich beispielhaft empfängt die Vorrichtung 100 gemäß diesem Ausführungsbeispiel ferner ein zweites Spektrumsignal 155, das einen sich von dem Teilbereich 130 unterscheidenden zweiten Teilbereich 153 des Interessenbereichs 140 durch eine hohe spektrale Auflösung aufweisende Spektraldaten abbildet, wobei die Bestimmeinrichtung 115 den Datensatz 150 unter Verwendung ferner des zweiten Spektrumsignals 155 bestimmt. Die Vorrichtung 100 ist ausgebildet, um entsprechend ein oder mehrere weitere Spektrumsignale zu empfangen, die je einen sich von dem Teilbereich 130 und dem zweiten Teilbereich 153 unterscheidenden anderen Teilbereich des Interessenbereich 140 durch die hohe spektrale Auflösung aufweisende Spektraldaten abbilden, wobei die Bestimmeinrichtung 115 dann den Datensatz 150 unter Verwendung ferner des weiteren Spektrumsignals oder der weiteren Spektrumsignale bestimmt.

Bei dem Interessenbereich 140 handelt es sich gemäß diesem Ausführungsbeispiel um einen Teil einer von einer oder mehreren Pflanzen 105 bewachsenen Agrarfläche, welche von der Sprüh- und Streuvorrichtung 110 besprüht wird oder werden soll. Das Bildsignal 125 repräsentiert ein Abbild des gesamten Interessenbereichs 140 und weist gemäß einem Ausführungsbeispiel eine hohe örtliche Auflösung auf. Das Spektrumsignal 145, 155 hingegen repräsentiert ein Abbild lediglich eines Teilbereichs 130, 153 des Interessenbereichs 140 und weist gemäß einem Ausführungsbeispiel gegenüber dem Bildsignal 125 eine geringere örtliche Auflösung auf.

Die Bilddaten mit der geringen spektralen Auflösung bilden gemäß diesem Ausführungsbeispiel einen ersten Wellenlängenbereich und zumindest einen zweiten Wellenlängenbereich ab, wobei sich der erste Wellenlängenbereich von dem zweiten Wellenlängenbereich unterscheidet. Beispielsweise umfassen die Bilddaten erste Intensitätswerte von dem ersten Wellenbereich zugeordneten Bildpunkten und zweite Intensitätswerte von dem zweiten Wellenbereich zugeordneten Bildpunkten. Der erste Wellenlängenbereich und der zweite Wellenlängenbereich umfassen gemäß diesem Ausführungsbeispiel Wellenlängenbereiche, welche von welche von Pflanzen reflektiert werden. Die geringe spektrale Auflösung ermöglicht typischerweise keine Erkennung einer kranken Pflanze oder Erkennung einer spezifischen Pflanzenkrankheit oder eines Nährstoffmangels. Der erste Wellenlängenbereich umfasst gemäß diesem Ausführungsbeispiel beispielsweise einen roten Spektralbereich von 630nm bis 680nm. Der zweite Wellenlängenbereich umfasst gemäß diesem Ausführungsbeispiel einen nahen Infrarotspektralbereich von 730nm bis 1100nm. Die Bilddaten mit der geringen spektralen Auflösung bilden gemäß einem Ausführungsbeispiel ferner auch einen dritten Wellenlängenbereich und/oder vierten Wellenlängenbereich ab, wobei sich der erste Wellenlängenbereich und der zweite Wellenlängenbereich von dem dritten und vierten Wellenlängenbereich unterscheiden. Die hohe spektrale Auflösung der Spektraldaten ist gemäß diesem Ausführungsbeispiel höher als die geringe spektrale Auflösung der Bilddaten. Die unter Verwendung der Vorrichtung 100 modifizierte spektrale Auflösung der Schätzdaten ist gemäß diesem Ausführungsbeispiel höher als die geringe spektrale Auflösung der Bilddaten oder weist sogar die hohe spektrale Auflösung der Spektraldaten auf. Unter Verwendung der Vorrichtung 100 wird gemäß diesem Ausführungsbeispiel ein Datensatz 150 erzeugt, der die hohe örtliche Auflösung des Bildsignals 125 aufweist und zumindest für Teilbereiche 130, 153 eine gegenüber dem Bildsignal 125 verbesserte spektrale Auflösung aufweist, um den Pflanzengesundheitszustand der Pflanzen 105 erkennbar zu machen.

Die Vorrichtung 100 weist ferner gemäß diesem Ausführungsbeispiel eine Spektraleinrichtung 160 mit zumindest einer hyperspektralen oder multispektralen Bildgebungskamera 165, einem Spektrometer und/oder einer Mehrzahl von LEDs mit Fotodioden auf, die ausgebildet ist, um das Spektrumsignal 145 und/oder zweite Spektrumsignal 155 zu erfassen und/oder für die Bestimmeinrichtung 115 bereitzustellen. Das Spektrumsignal 145 repräsentiert gemäß diesem Ausführungsbeispiel ein von der Spektraleinrichtung 160 aufgenommenes Bild oder Spektrum des Teilbereichs 130 und/oder das zweite Spektrumsignal 155 repräsentiert gemäß diesem Ausführungsbeispiel ein von der Spektraleinrichtung 160 aufgenommenes Bild oder Spektrum des zweiten Teilbereichs 153. Die Spektraleinrichtung 160 ist gemäß diesem Ausführungsbeispiel an einem Spritzgestänge der Sprüh- und Streuvorrichtung 110 angeordnet.

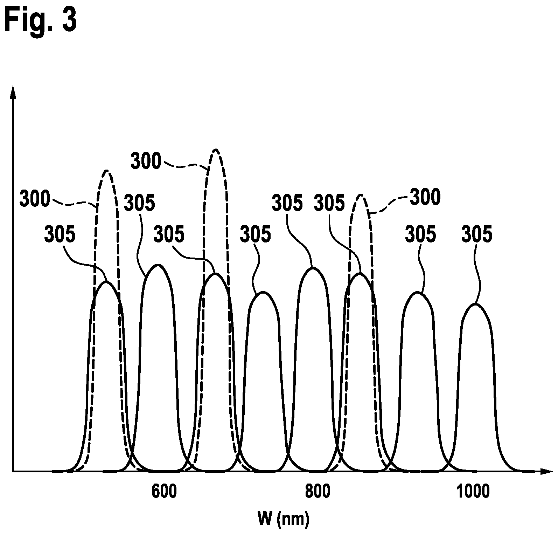

Die Bestimmeinrichtung 115 liest das Bildsignal 125 gemäß diesem Ausführungsbeispiel von einer Schnittstelle zu einer Kameraeinrichtung 170 mit einer ersten Kamera und/oder zumindest einer zweiten Kamera ein, wobei das Bildsignal 125 ein von der ersten Kamera aufgenommenes erstes Kamerabild und/oder ein von der zweiten Kamera aufgenommenes zweites Kamerabild repräsentiert. Die Kameraeinrichtung 170 ist gemäß diesem Ausführungsbeispiel an dem Spritzgestänge der Sprüh- und Streuvorrichtung 110 angeordnet. Die Kameraeinrichtung 170 weist gemäß diesem Ausführungsbeispiel einen optischen Filter auf, der lediglich Wellenlängen in einem roten Bandbereich, beispielsweise von 630nm bis 680nm, und/oder infraroten oder nahinfraroten Bandbereich, beispielsweise von 730nm bis 1100nm, durchlässt. Weitere optionale Merkmale der Kameraeinrichtung 170 werden in Fig. 3 beschrieben.

Gemäß einem Ausführungsbeispiel umfasst die Vorrichtung 100 ferner die Kameraeinrichtung 170 und/oder Sprüh- und Streuvorrichtung 110.

Die hier vorgestellte Vorrichtung 100 ermöglicht vorteilhafterweise eine Messung des Pflanzengesundheitsstatus für eine Sprüh- und Streuvorrichtung 110, die auch als Sprühsystem bezeichnet werden kann.

Die Kameraeinrichtung 170 umfasst gemäß diesem Ausführungsbeispiel zumindest eine Kamera mit 2,3mP (Megapixel) mit Filtern zur Messung von rotem Licht, kurz "R" oder "ROT", in einem Wellenlängenbereich von 660 nm und/oder nahinfrarotem Licht, kurz "NIR" in einem Wellenlängenbereich von 860 nm. Für die Aufnahme von Bildern umfasst die Kameraeinrichtung 170 ferner gemäß einem optionalen Ausführungsbeispiel eine aktive Beleuchtungseinrichtung mit LEDs oder mit kontinuierlichen Weißlichtquellen in den gleichen Bändern/relevanten Spektralbändern. Zusätzlich oder alternativ umfasst die Spektraleinrichtung 160 ferner gemäß einem Ausführungsbeispiel eine aktive Beleuchtungseinrichtung mit LEDs oder mit kontinuierlichen Weißlichtquellen in den jeweils gleichen Bändern/relevanten Spektralbändern eines optischen Filters der Spektraleinrichtung 160.

Der Kamera-ROI der Kameraeinrichtung 170 deckt gemäß diesem Ausführungsbeispiel den gesamten Bereich des Sprühens ab. Daten von der ersten und/oder zweiten Kamera der Kameraeinrichtung 170 sowie von der Spektraleinrichtung 160 werden gemäß diesem Ausführungsbeispiel von der Sprüh- und Streuvorrichtung 110 für die Spritzentscheidung im richtigen Bereich mit der richtigen Dosierung verwendet.

Das Reflexionsspektrum von Pflanzen 105 wird beeinflusst durch den Pflanzentyp, die Art der Ernährung, Wasserangebot, Stress, Schädlinge und Krankheiten. Pflanzenfarbe, Blattfarbe, Pflanzen-/Blattfarbstruktur, beispielsweise in Form einer speziellen Farbverteilung auf einem Blatt, und Blattkontur werden beeinflusst durch Pflanzenart, Pflanzensorte, Ernährungszustand, Wasserangebot, Stress, Schädlinge und Krankheiten. Gemäß diesem Ausführungsbeispiel ist die Vorrichtung 100 ausgebildet, um z. B. die Größe, Anzahl und Kontur der Pflanzen 105 auf Basis eines Normierten differenzierten Vegetationsindex, kurz "NDVI" (Normalized Difference Vegetation Index) mit hoher räumlicher Auflösung in der Größenordnung von mm pro Pixel auszuwerten. Die Vorrichtung 100 arbeitet hierzu vorteilhafterweise nicht nur mit zwei Wellenlängen, ROT 660 nm und NIR 860 nm, und berechnet den NDVI auf Basis dieser beiden Wellenlängen, sondern es werden für die Bewertung der Pflanzengesundheit und des Pflanzenernährungszustands typische zusätzliche Wellenlängendaten im sogenannten "roten Randbereich" zwischen 670 nm bis 770 nm verwendet, welche gemäß diesem Ausführungsbeispiel von der Spektraleinrichtung 160 bereitgestellt werden. Eine Auswertung des Pflanzengesundheits- und Pflanzenernährungszustandes wäre mit den zwei Wellenlängen ROT 660 nm und NIR 860 nm alleine schwierig zu realisieren. Die Vorrichtung 100 ist daher ausgebildet, um ein Referenzsystem zu verwenden, das zumindest das sogenannte "rote Randband" zwischen 670 bis 770nm an einzelnen Punkten mit geringer räumlicher Auflösung misst und diese spektrale Information mit dem bereits verwendeten System in Form der Kameraeinrichtung 170 mit höherer räumlicher Auflösung zu einem berechneten "Bild" mit hoher spektraler und spezieller Auflösung zu kombinieren. Damit ist eine Steuerung der landwirtschaftlichen Inputapplikation, z. B. Dünger, Wachstumsregulatoren, Fungizide, in einem präziseren und bedarfsgerechten Maß möglich.

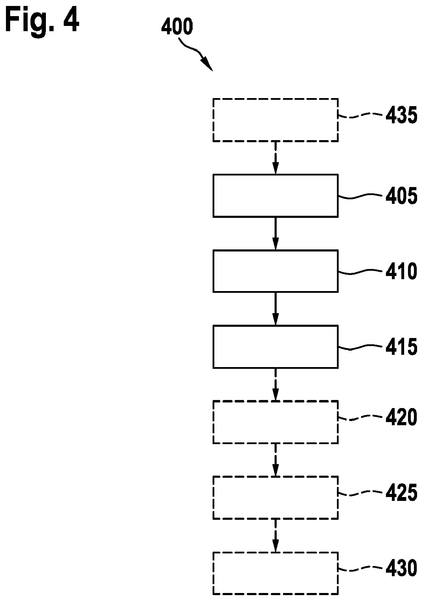

Die Vorrichtung 110 oder ein in Fig. 4 beschriebenes entsprechendes Verfahren realisiert eine Verwendung von zumindest einer Kamera oder mehreren Kameras einer Kameraeinrichtung 170 am Spritzgestänge mit nur zwei, drei oder vier Wellenlängen auf Basis von RGB- oder RGB-NIR-Kamera/s, z. B. 650nm, 850nm, mit Bilderkennung, die die meisten Flächen und Pflanzen 105 abdecken, die von der Düse 120 besprüht werden. Dies ist kombiniert mit der Verwendung von einer oder mehreren hyperspektralen oder multispektralen Bildgebungskameras 165, die nur an einigen spezifischen Punkten beispielsweise am Spritzgestänge montiert sind, mit einer oder mehreren Wellenlängen und gemäß einem Ausführungsbeispiel optional mindestens einer gleichen Wellenlänge wie eine der Kameras der Kameraeinrichtung 170, und die zumindest in einem spezifischen Bereich den gleichen Interessenbereich 140 abdecken wie die Kameraeinrichtung 170. Dies ist gemäß einem Ausführungsbeispiel kombiniert mit einem in Fig. 2 beschriebenen Verfahren zum Referenzieren der erfassten Interessenbereiche 140 der Kameraeinrichtung 170 mit dem der Spektraleinrichtung 160 mit mindestens einer Wellenlänge im gleichen Bereich, um eine Art Transferfunktion zu erhalten und/oder mit einem Verfahren zur Interpolation und Extrapolation eines Spektraleinrichtungs-Datensatzes von der Spektraleinrichtung 160 mit einem Kameraeinrichtungs-Datensatz der Kameraeinrichtung 170, um den Datensatz 150 über den gesamten von der Kameraeinrichtung 170 erfassten Bereich mit dem gesamten spektralen Erkennungsbereich von der Spektraleinrichtung 160 zu erhalten. Gemäß einem in Fig. 2 beschriebenen Ausführungsbeispiel wird der Datensatz 150 in Kombination mit weiteren Daten verwendet, z. B. in Kombination mit Pflanzenreflexionsspektren für bestimmte Pflanzen 105 und Pflanzengesundheitszustände, um den spezifischen Pflanzengesundheitszustand in dem Bereich, der von der Sprüh- und Streuvorrichtung 110 behandelt wird, zu identifizieren, zu berechnen oder zu approximieren. Gemäß einem in Fig. 2 beschriebenen Ausführungsbeispiel wird die Sprüh- und Streuvorrichtung 110 so angesteuert, dass auf der Grundlage dieses Datensatzes 150 die Art und/oder die Dosierung der Eingaben optimiert werden, um den besten oder vordefinierten Pflanzengesundheitszustand zu erhalten.

Gemäß einem alternativen Ausführungsbeispiel empfängt die Vorrichtung 110 das Spektrumsignal 145 und/oder zweite Spektrumsignal 155 von einem Spektrometer/Punktquellen und/oder von LEDs mit Fotodioden anstelle von hyperspektralen oder multispektralen Bildgebungskameras 165.

Gemäß einem Ausführungsbeispiel wird beispielsweise sowohl in den Bilddaten als auch in den Spektraldaten nach ein und derselben Pflanze 105 oder nach einem Blatt der Pflanze 105 gesucht. Dazu kann auf Verfahren der Objekterkennung zurückgegriffen werden. Bildpunkte in den Bilddaten und den Spektraldaten, die diese Pflanze 105 oder in entsprechender Weise ein Blatt der Pflanze 105 abbilden werden gemäß einem Ausführungsbeispiel dem Pflanzenbereich 151 zugeordnet.

Unter Verwendung einer örtlichen Transferfunktion werden die dem Pflanzenbereich 151 zugeordneten Bildpunkte der Bilddaten und der Spektraldaten referenziert. Die örtliche Transferunktion ist dabei beispielsweise für die Vorrichtung 100 vorbestimmt. Optional werden Parameter der örtlichen Transferfunktion für die Vorrichtung 100 in einem Kalibriervorgang bestimmt.

Jeder dem Pflanzenbereich 151 zugeordnete Bildpunkt der Bilddaten bildet ein Spektrum ab, das beispielsweise nur zwei oder drei Spektrallinien umfasst. Aufgrund der höheren spektralen Auflösung bildet jeder dem Pflanzenbereich 151 zugeordnete Bildpunkt der Spektraldaten ein Spektrum ab, das beispielsweise ebenfalls die von den Bildpunkten der Bilddaten abgebildeten Spektrallinien, zusätzlich jedoch noch weitere Spektrallinien umfasst. Unter Verwendung einer spektralen Transferfunktion werden die Spektren der dem Pflanzenbereich 151 zugeordneten Bildpunkte der Bilddaten um zumindest eine der weiteren Spektrallinien der Spektren der Bildpunkte der Spektraldaten ergänzt. Die spektrale Transferunktion ist dabei beispielsweise für die Vorrichtung 100 vorbestimmt. Optional werden Parameter der spektralen Transferfunktion für die Vorrichtung 100 in einem Kalibriervorgang bestimmt.

Fig. 2 zeigt eine schematische Darstellung einer Vorrichtung 100 gemäß einem Ausführungsbeispiel. Dabei kann es sich um die anhand von Figur 1 beschriebene Vorrichtung 100 handeln, die gemäß diesem Ausführungsbeispiel ferner eine Identifiziereinrichtung 205, eine Erzeugungseinrichtung 210 und/oder eine Ausgabeeinrichtung 215 aufweist.

Die Identifiziereinrichtung 205 ist ausgebildet, um den Pflanzengesundheitszustand 207 der Pflanze in dem Interessenbereich unter Verwendung des Datensatzes 150 zu identifizieren. Hierbei wird der Pflanzengesundheitszustand 207 gemäß einem Ausführungsbeispiel ferner unter Verwendung von Pflanzenreferenzdaten 220 identifiziert. Beispielsweise wird der Pflanzengesundheitszustand 207 der Pflanze im Interessenbereich unter Verwendung der Pflanzenreferenzdaten 220 identifiziert, die Pflanzenreflexionsspektren für bestimmte Pflanzen und/oder Pflanzengesundheitszustände umfassen. Gemäß einem Ausführungsbeispiel verwendet die Identifiziereinrichtung 205 hierzu eine Nachschlagetabelle 225 mit hinterlegten Pflanzenreflexionsspektren für bestimmte Pflanzen und zugeordneten Pflanzengesundheitszuständen, um den Pflanzengesundheitszustand 207 schnell und einfach durch Zuordnung zu identifizieren. Gemäß einem Ausführungsbeispiel erkennt die Identifiziereinrichtung 205 ferner eine Blattkontur und/oder Farbverteilung der Pflanze unter Verwendung des Bildsignals 125 und/oder identifiziert den Pflanzengesundheitszustand 207 der Pflanze unter Verwendung der erkannten Blattkontur und/oder erkannten Farbverteilung der Pflanze.

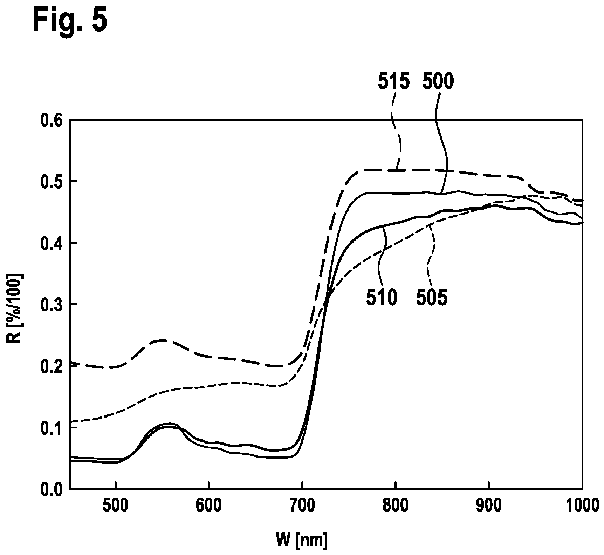

Beispielsweise wird gemäß einem Ausführungsbeispiel von der Identifiziereinrichtung 205 ein Stickstoffmangel einer Pflanze durch eine hellgrüne oder gelbe Blattfarbe, ein Phosphormangel durch eine rötlich bis purpurfarbene Blattverfärbung, ein Kaliummangel durch trockene Blattränder, ein Magnesiummangel durch weiße Streifen entlang der Blattadern, Herbizidschäden durch verbrannte Blattspitzen und/oder -ecken sowie weiße Verfärbungen, Trockenstress durch graugrüne sowie eingerollte Blätter und/oder die Blattfleckenkrankheit durch kleine Flecken auf den Blättern der Pflanze identifiziert. Verschiedene Spektralsignaturen von gesunden und kranken Zuckerrübenblättern sind in Fig. 5 gezeigt, derartige Spektralsignaturen und/oder vergleichbare andere Spektralsignaturen für andere Pflanzen sind gemäß einem Ausführungsbeispiel als die Pflanzenreferenzdaten 220 in der Nachschlagetabelle 225 hinterlegt.

Die Erzeugungseinrichtung 210 ist ausgebildet, um unter Verwendung des Datensatzes 150 ein Einstellsignal 230 zum Einstellen einer Art und/oder Dosierung eines Sprühmittels und/oder Streumittels der Sprüh- und Streuvorrichtung 110 zum Behandeln des Interessenbereichs 140 zu erzeugen. Die Erzeugungseinrichtung 210 erzeugt das Einstellsignal 230 gemäß diesem Ausführungsbeispiel ferner unter Verwendung des von der Identifiziereinrichtung 205 identifizierten Pflanzengesundheitszustands 207.

Die Ausgabeeinrichtung 215 ist ausgebildet, um unter Verwendung der Sprüh- und Streuvorrichtung 110 und/oder des Einstellsignals 230 die von der Erzeugungseinrichtung 210 eingestellte Art und/oder Dosierung des Sprühmittels auszugeben. Die Ausgabeeinrichtung 215 ist gemäß diesem Ausführungsbeispiel Teil der Düse 120.

Die Bestimmeinrichtung 115 ist gemäß diesem Ausführungsbeispiel ausgebildet, um den Datensatz des Pflanzenbereichs unter Verwendung zumindest einer Transferfunktion 235 aus dem Bildsignal 125 und dem Spektrumsignal 145 zu bestimmen. Die Transferfunktion 235 wird gemäß einem Ausführungsbeispiel als hinterlegte, beispielsweise zuvor bestimmte, Transferfunktion 235 eingelesen und/oder angewandt. Die Bestimmeinrichtung 115 ist gemäß diesem Ausführungsbeispiel ausgebildet, um unter Verwendung der zumindest einen Transferfunktion 235 ein von den Bilddaten für den Pflanzenbereich abgebildetes Spektrum um zumindest einen Spektralbereich eines von den Spektraldaten für den Pflanzenbereich abgebildeten Spektralbereich zu ergänzen. Die Bestimmeinrichtung 115 ist gemäß einem Ausführungsbeispiel ausgebildet, um die Spektraldaten zu dem Bilddaten unter Verwendung einer örtlichen Transferfunktion zu referenzieren, um eine räumliche Zuordnung zwischen den Spektraldaten und den Bilddaten zu erhalten. Beispielsweise referenziert die Bestimmeinrichtung 115 hierbei gemäß einem Ausführungsbeispiel die Spektraldaten zu den Bilddaten, indem Bildintensitäten der Spektraldaten zu Bildintensitäten der Bilddaten referenziert werden. Hierbei wird beispielsweise eine Bildintensität einer bestimmten Wellenlänge oder einer Kombination aus Bildintensitäten einer Mehrzahl von Wellenlängen der Spektraldaten zu einer Bildintensität einer bestimmten Wellenlänge oder einer Kombination aus Bildintensitäten einer Mehrzahl von Wellenlängen der Bilddaten referenziert. Anders ausgedrückt wird eine Referenzierung gemäß einem Ausführungsbeispiel unter Verwendung einer Intensität in einem speziellen Spektralband oder einer Wellenlänge in einem Teilbereich der ROI und/oder einer räumlichen Verteilung von Referenzobjekten in der ROI, beides und im Falle von zwei oder mehr Wellenlängen durch Verhältnisse/Proportionen von zwei oder mehr Wellenlängen und Kombinationen durchgeführt. Optional wird gemäß einem Ausführungsbeispiel eine Interpolation und Extrapolation durchgeführt, um beispielsweise weitere Schätzdaten für jene Teilbereiche des Interessenbereichs zu erhalten, für welche kein Spektrumsignal zur Verfügung steht. Die Bestimmeinrichtung 115 ist gemäß diesem Ausführungsbeispiel ferner ausgebildet, um die Spektraldaten zu den Bilddaten unter Verwendung einer spektralen Transferfunktion zu referenzieren, um eine spektrale Zuordnung zwischen den Spektraldaten und den Bilddaten zu erhalten. Die Bestimmeinrichtung 115 ist gemäß einem Ausführungsbeispiel ferner ausgebildet, um die Spektraldaten zu den Bilddaten unter Verwendung zumindest einer Spektrallinie zu referenzieren, die sowohl von den Spektraldaten als auch von den Bilddaten abgebildet wird.

Die Vorrichtung 100 ist ferner gemäß einem anderen Ausführungsbeispiel ausgebildet, um ein Referenzsignal 240 von einem Referenzsensor 245 zu empfangen, um die Transferfunktion 235 zu bestimmen. Das Referenzsignal 240 dient gemäß einem Ausführungsbeispiel zur Analyse und Berücksichtigung der Intensität, der räumlichen und winkelmäßigen Verteilung, der spektralen Verteilung des Umgebungslichts, z. B. des Sonnenlichts, in der Transferfunktion 235 und Bilderkennung der als hyperspektrale oder multispektrale Bildgebungskamera/s ausgeformten Spektraleinrichtung. Der Referenzsensor 245 ist gemäß einem Ausführungsbeispiel z. B. auf einem Kabinendach des landwirtschaftlichen Geräts/Schleppers, welches/r die Sprüh- und Streuvorrichtung 110 aufweist, oder an der Düse 120 der Sprüh- und Streuvorrichtung 110 angeordnet. Gemäß einem Ausführungsbeispiel ist die Vorrichtung 100 zusätzlich oder alternativ ausgebildet, um ein spezifisches Testziel im Bereich der Kameras der Kameraeinrichtung und der Spektraleinrichtung als Methode zur Referenzierung der erfassten ROIs von der Kameraeinrichtung mit der Spektraleinrichtung zu verwenden, um die Art der Transferfunktion 235 zu erhalten.

Fig. 3 zeigt eine schematische Darstellung von Bilddaten und Spektraldaten zur Verwendung mit einer Vorrichtung gemäß einem Ausführungsbeispiel. Dabei kann es sich um eine der anhand der vorangegangenen Figuren beschriebenen Vorrichtungen handeln. Auf der x-Achse sind Wellenlängen W in nm und auf der y-Achse eine Signalintensität dargestellt.

Dargestellt sind erste Spektrallinien 300 der Bilddaten mit geringer spektraler Auflösung sowie zweite Spektrallinien 305 der Spektraldaten mit der höheren spektralen Auflösung. Die Bestimmeinrichtung referenziert gemäß einem Ausführungsbeispiel die Spektraldaten zu den Bilddaten unter Verwendung zumindest einer der Spektrallinien 300, 305, die sowohl von den Spektraldaten als auch von den Bilddaten abgebildet wird.

Fig. 4 zeigt ein Ablaufdiagramm eines Verfahrens 400 zum Erkennen eines Pflanzengesundheitszustands von Pflanzen für eine Landmaschine gemäß einem Ausführungsbeispiel. Dabei kann es sich um ein Verfahren 400 handeln, das von einer der anhand der vorangegangenen Figuren beschriebenen Vorrichtungen ausführbar ist.

Das Verfahren 400 umfasst einen Schritt 405 des Einlesens, einen Schritt 410 des Empfangens und einen Schritt 415 des Bestimmens. Im Schritt 405 des Einlesens wird ein Bildsignal eingelesen, das einen eine Mehrzahl von Teilbereichen umfassenden Interessenbereich abbildet, wobei jedem Teilbereich ein eine geringe spektrale Auflösung aufweisende Bilddaten zugewiesen sind. Im Schritt 410 des Empfangens wird ein Spektrumsignal empfangen, das einen der Teilbereiche des Interessenbereichs durch eine hohe spektrale Auflösung aufweisende Spektraldaten abbildet. Im Schritt 415 des Bestimmens wird ein Datensatz bestimmt, der den die Mehrzahl von Teilbereichen umfassenden Interessenbereich abbildet, wobei jedem Teilbereich eine gegenüber der geringen spektralen Auflösung modifizierte spektrale Auflösung aufweisende Schätzdaten zugewiesen ist, unter Verwendung des Bildsignals und des Spektrumsignals.

Optional umfasst das Verfahren 400 gemäß diesem Ausführungsbeispiel ferner einen Schritt 420 des Identifizierens, einen Schritt 425 des Erzeugens, einen Schritt 430 des Ausgebens und/oder einen Schritt 435 des Erfassens.

Im Schritt 420 des Identifizierens wird der Pflanzengesundheitszustand der Pflanze in dem Interessenbereich unter Verwendung des Datensatzes identifiziert. Im Schritt 425 des Erzeugens wird ein Einstellsignal zum Einstellen einer Art und/oder Dosierung eines Sprühmittels und/oder Streumittels einer Sprüh- und Streuvorrichtung zum Behandeln des Interessenbereichs unter Verwendung des Datensatzes erzeugt. Im Schritt 430 des Ausgebens wird unter Verwendung der Sprüh- und Streuvorrichtung und/oder des Einstellsignals die im Schritt 425 des Erzeugens eingestellte Art und/oder Dosierung des Sprühmittel und/oder Streumittels ausgegeben. Im Schritt 435 des Erfassens wird das Spektrumsignal unter Verwendung einer Spektraleinrichtung mit zumindest einer hyperspektralen oder multispektralen Bildgebungskamera, einem Spektrometer und/oder einer Mehrzahl von LEDs mit Fotodioden erfasst.

Fig. 5 zeigt Spektralsignaturen von gesunden Blättern 500 und kranken Blättern einer Rübenpflanze. Auf der x-Achse sind Wellenlängen W in nm und auf der y-Achse die Reflexionen R in Prozent dargestellt. Gezeigt sind neben der Spektralsignatur von gesunden Blättern 500, jene Spektralsignaturen von Blättern, die je von der Cercospora-Blattfleckenkrankheit 505, Zuckerrübenrost 510 und echtem Mehltau 515 befallen sind. Die Reflexionsspektren stellen den Mittelwert von n = 20 Pixeln des charakteristischen Gewebes dar.

Fig. 6 zeigt eine schematische Darstellung einer Landmaschine 600 mit einer Vorrichtung 100 gemäß einem Ausführungsbeispiel. Dabei kann es sich um die anhand von einer der vorangegangenen Figuren beschriebene Vorrichtung 100 handeln.

Die Sprüh- und Streuvorrichtung 110 ist gemäß diesem Ausführungsbeispiel an der Landmaschine 600 angeordnet.

Für die Aufnahme von Bildern umfasst die Vorrichtung 100 oder Kameraeinrichtung 170 ferner gemäß diesem Ausführungsbeispiel eine aktive Beleuchtungseinrichtung 605 mit kontinuierlichen Weißlichtquellen oder LEDs in den gleichen Bändern/relevanten Spektralbändern eines optischen Filters der Kameraeinrichtung 170.

## Patentansprüche

1. Verfahren (400) für eine Landmaschine (600) zum Erkennen eines Pflanzengesundheitszustands (207) von Pflanzen (105), wobei das Verfahren (400) die folgenden Schritte umfasst:
Einlesen (405) eines Bildsignals (125), das einen zumindest einen Teilbereich (130) umfassenden Interessenbereich (140) abbildet, wobei dem Interessenbereich (140) eine geringe spektrale Auflösung aufweisende Bilddaten zugewiesen sind;
Empfangen (410) eines Spektrumsignals (145), das den Teilbereich (130) des Interessenbereichs (140) durch eine hohe spektrale Auflösung aufweisende Spektraldaten abbildet; und
Bestimmen (415) eines Datensatzes (150), der zumindest einen von dem Teilbereich (130) umfassten Pflanzenbereich (151) abbildet, wobei dem Pflanzenbereich (151) eine gegenüber der geringen spektralen Auflösung modifizierte spektrale Auflösung aufweisende Schätzdaten zugewiesen sind, unter Verwendung des Bildsignals (125) und des Spektrumsignals (145).

2. Verfahren (400) gemäß Anspruch 1, bei dem im Schritt (415) des Bestimmens der Datensatz (150) des Pflanzbereichs unter Verwendung zumindest einer Transferfunktion (235) aus dem Bildsignal (125) und dem Spektrumsignal (145) bestimmt wird.

3. Verfahren (400) gemäß Anspruch 2, bei dem im Schritt des Bestimmens unter Verwendung der zumindest einen Transferfunktion ein von den Bilddaten für den Pflanzenbereich (151) abgebildetes Spektrum um zumindest einen Spektralbereich eines von den Spektraldaten für den Pflanzenbereich (151) abgebildeten Spektralbereich ergänzt wird.

4. Verfahren (400) gemäß einem der Ansprüche 2 bis 3, bei dem im Schritt (415) des Bestimmens die Spektraldaten zu den Bilddaten unter Verwendung einer örtlichen Transferfunktion referenziert werden, um eine räumliche Zuordnung zwischen den Spektraldaten und den Bilddaten zu erhalten.

5. Verfahren (400) gemäß einem der Ansprüche 2 bis 4, bei dem im Schritt (415) des Bestimmens die Spektraldaten zu den Bilddaten referenziert werden, indem Bildintensitäten der Spektraldaten zu Bildintensitäten der Bilddaten referenziert werden.

6. Verfahren (400) gemäß einem der Ansprüche 2 bis 5, bei dem im Schritt (415) des Bestimmens die Spektraldaten zu den Bilddaten unter Verwendung einer spektralen Transferfunktion referenziert werden, um eine spektrale Zuordnung zwischen den Spektraldaten und den Bilddaten zu erhalten.

7. Verfahren (400) gemäß einem der Ansprüche 2 bis 6, bei dem im Schritt (415) des Bestimmens die Spektraldaten zu den Bilddaten unter Verwendung zumindest einer Spektrallinie (300, 305) referenziert werden, die sowohl von den Spektraldaten als auch von den Bilddaten abgebildet wird.

8. Verfahren (400) gemäß einem der vorangegangenen Ansprüche, mit einem Schritt (420) des Identifizierens, in dem der Pflanzengesundheitszustand (207) der Pflanze (105) in dem Interessenbereich (140) unter Verwendung des Datensatzes (150) identifiziert wird.

9. Verfahren (400) gemäß Anspruch 8, bei dem im Schritt (420) des Identifizierens der Pflanzengesundheitszustand (207) ferner unter Verwendung von Pflanzenreferenzdaten (220) identifiziert wird.

10. Verfahren (400) gemäß Anspruch 9, bei dem im Schritt (420) des Identifizierens der Pflanzengesundheitszustand (207) der Pflanze (105) im Interessenbereich (140) unter Verwendung der Pflanzenreferenzdaten (220) identifiziert wird, die Pflanzenreflexionsspektren für bestimmte Pflanzen und/oder Pflanzengesundheitszustände umfassen.

11. Verfahren (400) gemäß einem der vorangegangenen Ansprüche, mit einem Schritt (425) des Erzeugens eines Einstellsignals (230) zum Einstellen einer Art und/oder Dosierung eines Sprühmittels und/oder Streumittels einer Sprüh- und Streuvorrichtung (110) zum Behandeln des Interessenbereichs (140) unter Verwendung des Datensatzes (150).

12. Verfahren (400) gemäß Anspruch 11, mit einem Schritt (430) des Ausgebens, in dem unter Verwendung der Sprüh- und Streuvorrichtung (110) und/oder des Einstellsignals (230) die im Schritt (425) des Erzeugens eingestellte Art und/oder Dosierung des Sprühmittel und/oder Streumittels ausgegeben wird.

13. Verfahren (400) gemäß einem der vorangegangenen Ansprüche, mit einem Schritt (435) des Erfassens, in dem das Spektrumsignal (145) unter Verwendung einer Spektraleinrichtung (160) mit zumindest einer hyperspektralen oder multispektralen Bildgebungskamera (165), einem Spektrometer und/oder einer Mehrzahl von LEDs mit Fotodioden erfasst wird.

14. Verfahren (400) gemäß einem der vorangegangenen Ansprüche, bei dem im Schritt (405) des Einlesens das Bildsignal (125) von einer Schnittstelle zu einer Kameraeinrichtung (170) mit einer ersten Kamera und/oder zumindest einer zweiten Kamera eingelesen wird, wobei das Bildsignal (125) ein von der ersten Kamera aufgenommenes erstes Kamerabild und/oder ein von der zweiten Kamera aufgenommenes zweites Kamerabild repräsentiert.

15. Vorrichtung (100), die eingerichtet ist, um die Schritte (405, 410, 415, 420, 425, 430, 435) des Verfahrens (400) gemäß einem der vorangegangenen Ansprüche in entsprechenden Einheiten (110, 115, 160, 165, 170, 205, 210, 215) auszuführen und/oder anzusteuern.

16. Computerprogrammprodukt, insbesondere Datenprogramm oder Datenträger, mit einem Algorithmus, der dazu ausgebildet ist, ein Verfahren nach einem der Ansprüche 1 bis 14 auszuführen, wenn dieses auf einem Computer ausgeführt wird.
